# EUROPEAN PATENT SPECIFICATION

(11) **EP 1 911 412 B1**
(45) Date of publication and mention of the grant of the patent: **13.04.2011**
(21) Application number: 06122075.2
(22) Date of filing: 11.10.2006
(51) Int. Cl.: A61C 8/00

(54) **Implant**
Implantat
Implant

(43) Date of publication of application: 16.04.2008
(73) Proprietor: Astra Tech AB, 431 21 Mölndal (SE)
(72) Inventor: Hansson, Stig, 436 39 Askim (SE); Aringskog, Per, 43020 Veddige (SE); Holmström, Johan, 42837 Kållered (SE)
(74) Representative: Vink, Charlotta

(56) References cited:
- WO-A-2006/049385
- WO-A-2007/048267
- FR-A1- 2 855 391
- JP-A- 9 135 844
- US-A- 5 823 777
- US-A- 5 902 109
- US-A- 6 129 730
- US-A1- 2004 091 837

## Description

### Field of the invention

The present invention relates to an implant, an implant set, and a method of implanting an implant. The implant comprises an external fixture thread having a radius, thread making means, alternatively a separate thread making means, at an apical end of the external thread for making a bone thread in bone tissue for insertion of the implant.

### Technical background

A frequent way today to restore a damaged limb, such as lost tooth, damaged hip joint or finger, is to install an implant in the adjacent bone tissue and replace the damaged parts. In this respect it is of vital importance for a successful result that the implant becomes fully stable and is correctly joined to the bone. The term osseointegration is used for this joining effect, the basic meaning of this term being the bone tissue growth into the implant surface. The two major contributors to this joint are a mechanical joint and an organic joint. The former being generally influenced by the macro geometry of the bore into which the implant is installed, and by the macro geometry of the implant, and is a direct effect of how well these two work together. The latter one being a continuously evolving and developing effect, particularly the time immediately after installation, and being generally influenced by how well the micro surface structure of the implant and its surface chemistry adheres to the bone tissue.

Due to ingrowth there will be an interlocking effect between the bone and the implant. Also, the mechanical joint is developed over time since the bone tissue, under ideal conditions, may grow into surface cavities of the implant, and grow into voids left between the implant and the bore after installation.

Interaction of mechanic and organic aspects will affect the ingrowth and joint between the bone and the implant threads. Further a blasted surface of the implant will provide advantageous conditions for this process.

One of the major problems relating to successful installation of implants in bone tissue of a patient is the effect of bone resorption around the implant. This means that the bone tissue in the close proximity of the lateral surface of the implant after implant installation starts to regress or resorb due to necrosis or death of the living bone tissue. This in turn leads to a poorer stability of the implant, since the implant is not surrounded by as much bone tissue as was intended.

Under certain conditions the bone tissue may later grow back, and hence, at least partly recover the stability of the implant. The bone tissue may - and the implant is often designed to - also grow further towards the implant than the initially drilled hole to further improve the long-term stability of the implant.

Especially when the implant is intended to be subject to early loading it is vital that the stability directly after implantation is kept intact. Due to bone resorption surgeons have been necessitated to take the effect into consideration when designing implantation systems and when planning a procedure on a patient. The emphasis has so far been placed upon adaptation to the effect rather than fully overcoming it.

Document US 5 902 109 A discloses an implant with an external fixture thread and thread making means at an apical end of said external thread for making a bone thread in bone tissue during insertion of the implant. Said thread making means having a radius, which radius is generally equal to the radius of a first portion of the external fixture thread, wherein the radius of a second portion of said external fixture thread is reduced in relation to said radius of the thread making means.

In SE 516917 is shown an implant with non-round outer geometry. The purpose of this solution is to provide an increased pressing of the bone tissue to solve the problem with initial stability, especially in soft or weak bone tissue. Further it is of high importance to combine the non-round geometry with a conical implant according to this document. The implant with design according to SE 516917 is meant to provide good stability with regard to rotation after in-growth of the bone tissue towards the implant.

The document WO 2004/058091 discloses an implant provided with grooves, which are intended for increase of the surface of the thread flanks exposed to the bone. For improvement of bone growth a bone-growth-stimulating agent (TS or HA gel) is proposed.

Further WO 2004/098442 discloses an implant provided with a groove pattern intended for increasing the contact surface between the bone and the implant. An advantageous embodiment is shown having a groove pattern directed crosswise the extension of the implant thread, which is said providing an improved distribution of pressure.

In the publication "Understanding Peri-Implant Edosseous Healing" by John E. Davis the phenomenon of distance and contact osteogenesis are described. Further bone growth is described regarding three different phenomena; osteoconduction, de novo bone formation, and bone remodeling. The access for blood to the bone growth area is mentioned as an important mechanism.

It has been suggested that the necrosis is affected by the altered loading condition in the bone tissue due to the implant. Other reasons could be the trauma caused by the drilling of a hole for the implant and the heat produced thereby. A further effect that has been suggested, but which may not as much be affected by the surgeon, is the hormone balance of the patient. No one has however so far been able to fully explain the mechanisms behind the necrosis and tissue resorption, or later re-growth. The effects have nevertheless been studied and are well documented.

It is thus an object of the present invention to improve stability of an implant, in particular it is an object to improve stability some time after installation in the event of bone resorption. An implant according to the present invention thus presents stability all the time from installation into the bone tissue of a patient.

### Summary of the invention

It is an object of the present invention to provide an implant that addresses the above-mentioned issues. This is achieved by implants and implant sets according to the appended claims.

A first aspect of the invention is disclosed in claim 1.

"Radius" is in the concept of the present invention not limited to the distance from the centre axis to the top radius of a thread. The expression "radius" is used to define the distance between the centre axis and any position on a thread surface, since generally with the present invention, corresponding parts of the implant thread and the thread making means are compared, rather than sizes of different implants.

Thread making means could for instance be a tapping means, cutting means or a thread with a shape such that bone is pressed to form threads. However, generally within the present invention, tapping means or cutting means are preferred to threads pressing out bone threads.

The portion of the implant with reduced radius can be provided in many different ways e.g. as patterns along the implant, ring shaped areas around the implant, spot-shaped areas, spiral-shaped areas etc. One of the purposes with the reduced areas/portions is to provide a zone at the implant where the ingrowth is stimulated in a different cycle over time compared to the non-reduced area/portion. Hence, this reduced area/portion cannot be too small due to access of blood and further it is desirable to reduce pressure and stress at the reduced portion to provide a satisfactory bone growth. The same desired effect according to the inventive concept of the invention is achieved by providing an enlarged part/portion at the thread making portion, and the in this case the whole implant thread may have the same radius but smaller relative the thread making portion at least in some parts of the thread. The resulting effect is the same as with reduced portions, i.e. there will be areas/portions with no or reduced contact, pressure, stress between the implant and the bone, which will provide a positive influence on the bone growth.

However the desired ingrowth effect can be achieved by reducing or eliminating stress on the bone in the area of the reduced portions. Since bone is a relatively flexible material, it is possible that the bone in the area of the reduced portion does not leave a gap to the implant, but instead flex towards the implant providing an area of reduced stress.

The position of the reduced areas/portions is arranged to increase the stability/anchoring of the implant in the bone over time and further to provide an initially stimulated bone growth in the reduced area/portion. The arrangement of the reduced portions are hence preferably provided in such a way that both initial stable contact between the implant and the bone is achieved and secondary stable contact between the implant and the bone at the reduced portions is achieved after a period of time due to ingrowth, at the same time there may be resorption of the bone in the non-reduced areas.

During a third period of time, future stable contact between the implant and bone is achieved due to complete ingrowth of bone tissue with major portions/areas of the implant.

According to the inventive concept of the present invention, areas of variable pressure/distance between the implant and the bone tissue is achieved providing zones with different phases in the ingrowth cycle, and hence a firm engagement between the implant and the bone tissue over time, already from the installation of the implant. The non-reduced area/portion will engage the bone thread directly during installation. In the event of bone resorption some time after installation in the non-reduced area, the bone at the reduced areas will have reformed and can thus unload other areas with possible bone-resorption. This will provide an advantageous possibility to load the implant immediately after installation, and as a result of the reduced portion it is possible to continuously load the implant over time. The inventive concept does also have an advantageous effect on bone of poor quality, or rather, no matter the quality of the bone.

According to the invention, the portions with reduced radius are provided repeatedly at said implant for providing both initial stability and future stability between the implant and the bone due to the ingrowth process of the bone tissue with said implant.

The reduced portions can be provided cyclically/periodically along the implant with areas formed along the length of the implant to achieve stability between the implant and the bone due to different time cycles of ingrowth to different portions of the implant over time.

The reduced radius in relation to said radius of the thread making means is preferably 0-5%, more preferably 0-2,4% and most preferably 0,06-0,16% of the radius of the thread making means.

In some embodiments the bone tissue is preferably cut by tapping means, and hence not pressed, compressed or squeezed etc. A good effect is achieved if the area of the bone corresponding to the reduced portion of the implant is cut or sheared, normally with a tapping means having a cutting angle of 90° to the bone tissue, but can also be less than 90° provided that the thread is cut/tapped in the bone tissue and not pressed.

Further, the reduction of the thread is preferably such that the distance between a specific position of the thread and the closest corresponding position of the thread making means or adjacent bone is preferably 0-100µm, more preferably 0-50µm and most preferably 10-30µm. The distance can also be described as the distance between a specific position at a portion of the implant thread reduced relative the thread in the bone and the closest position in the bone when the implant is in its final position immediately after screwing the implant into the jawbone of a patient. Further the thread profile radius forming the bone thread, which is provided adjacent to the non-reduced threads of the implant, is generally equal to the thread profile radius of this first portion of the implant. This provides an even distribution of pressure at this portion of the bone tissue.

According to embodiments of the present invention, the crest of the second portion of said external fixture thread of said implant is reduced; alternatively, the bottom, an apical or coronal flank of the second portion of said external fixture thread of said implant is reduced. The radius of the flank may be reduced to the same extent as the crest, when measured perpendicular to a plane of extension of the flank.

The reduction may be provided extending continuously from said apical flank via said crest to said coronal flank, or other combinations of reduced flank/s, crest, and thread bottom or root of thread.

According to a further aspect of the present invention, the reduction of the fixture thread is equal as seen along the axial extension of the implant. In an alternative embodiment the core of the external fixture thread is provided with a reduced radius.

It may be advantageous if the implant, comprises a double external fixture thread, each of the thread profiles having a radius, and further comprising a thread making means at an apical end of the external fixture thread for making bone threads in bone tissue during insertion of the implant. The thread making means has a radius being generally equal to at least one external fixture thread radius, wherein the radius of one of said external fixture threads is reduced in relation to said radius of the thread making means. This provides, over time, either of the threads to be in engaging contact with the bone, and hence there will be a stable connection between the implant and the bone due to interaction between the double fixture thread and the bone tissue.

The reduced thread provides repeatedly reduced and non reduced threads seen along the length of said implant which provides both initial stability and future stability between the implant and the bone due to the ingrowth process of the bone tissue with said implant.

According to an aspect of the invention the radius of one of the external fixture threads is reduced in relation to corresponding radius of the thread making means. Of course it is also possible to combine implants with double or multiple thread and portions/areas with reduced radius for achieving the effect according to the invention.

It may be advantageous if the radius of said fixture thread is continuously reduced at portions along the length of the thread of said implant. This continuous reduction can be performed on the bottom/root, crest, apical flank, or coronal flank of said reduced external fixture thread or alternatively combinations of these portions of the thread.

It may be advantageous if the implant, comprises an external fixture thread having a radius, a reduced portion defined by a sector of the implant cross-section of less than 360°, and a thread making means at an apical end of the external fixture thread for making a bone thread in bone tissue during insertion of the implant. The thread making means has a radius being generally equal to the external fixture thread radius, wherein the radius of said reduced portion is reduced in relation to the radius of the thread making means.

Said second portion of the implant is constituted of a reduced portion/area of the external fixture thread of the implant. The transition from reduced portion to non-reduced portion may be gradually changed from reduced radius to generally equal radius.

According to aspects of the present invention, the reduced portion may have a curved or twisted axial extension along said implant.

According to an embodiment which is not part of the present invention the implant comprises two reduced portions, each reduced portion being defined by a sector of less than 180° of the cross-section of the implant. Further two or more reduced portions may be uniformly distributed around the implant, for providing continuous stability (over time) between the implant and the bone due to interaction between the external fixture thread and said reduced area, as a result of the different phases/stages of bone growth over time.

Preferably the reduced portions are positioned diametrically opposed one another, or positioned periodically for providing stability over time, due to ingrowth and resorption of the bone tissue adjacent said implant. Each reduced portion may have an axial extension along generally the entire length of the portion of the implant provided with external fixture thread.

It may further be possible to have an implant having a double or multiple external fixture thread, where a thread making means is provided with double or multiple thread making means having a first radius being generally equal to the corresponding external fixture thread radius, and further the thread making means having a second radius being increased (extended, enlarged, expanded) in relation to the corresponding external fixture thread radius.

A second aspect of the invention is disclosed in claim 13.

The thread making means can be shaped with a straight, tapering or conical profile, i.e. seen along the axis of the implant. It is usually the largest radius of the thread making means that forms the bone thread, and should be compared to the radius of said external fixture thread. This applies to the thread making means both if it is a separate thread making means or if it is an integrated thread making means.

The reduced radius of the implant compared to the radius of a separate thread making means is preferably 0-5%, more preferably 0-2,4% and most preferably 0,06-0,16% of the radius of the thread making means.

The reduction of the thread is preferably such that the distance between a specific position of the thread and the closest corresponding position of the thread making means is 0-100µm, more preferably 0-50µm and most preferably 10-30µm.

Preferably a bottom/root, crest, apical or coronal flank of said external fixture thread of said implant is reduced or alternatively combinations thereof. The radius of the root, flank and/or crest is preferably reduced to the same extent, when measured perpendicular to a plane of extension of the flank. This will provide a gap having a substantially uniform distance between the implant and the bone. Different portions of the thread profile are reduced to achieve the desired effect according to the inventive concept.

The reduction may be provided extending continuously from said root via the apical flank via the crest to said coronal flank, or other alternative combinations of reduced root, flank or crest of thread.

In a further alternative embodiment the implant set comprises an implant having a double external fixture thread, each of the thread profiles having a radius, the set further comprises a thread making means for making bone threads in bone tissue prior to insertion of said implant, said thread making means having a radius being generally equal to at least one external fixture thread radius, wherein the radius of one of said external fixture threads is reduced in relation to said radius of the thread making means.

The implant set can further be performed as; comprising an implant, a thread making means for making a bone thread in bone tissue prior to insertion of said implant, said implant comprising an external fixture thread having a radius, a first portion defined by a sector of the implant cross-section of less than 360°, said thread making means having a radius being generally equal to the radius of a first portion of said external fixture thread, wherein the radius of a second portion of said external fixture thread is reduced in relation to the radius of said thread making means.

The first portion is also provided with said external fixture thread. Suitably the radius changes gradually from generally equal radius to reduced radius.

The second portion may have a curved or twisted axial extension along said implant.

According to one embodiment, the implant is provided with two second or more portions, each second portion being defined by a sector of less than 180° of the cross-section of the implant. These two or more sectors are preferably uniformly distributed around the implant.

According to one aspect of the invention, the implant is a dental implant and is thus meant to be implanted in the jaw bone of a patient by dental surgeons.

Preferably, a method of implanting an implant into bone, comprises:
- boring a hole in the bone; and
- inserting into the bored hole of the bone an implant according to any one of the above-mentioned aspects or embodiments.

Suitably, the method may further comprise inserting the implant and screwing the implant into the bored hole.

The method may further comprise inserting the implant and making bone threads within the bored hole in the bone prior to said insertion of the implant using a thread making means. In this case the making of bone threads is performed with a separate thread making means prior to screwing the implant into the hole.

### Brief description of the figures

Preferred embodiments of the present invention will in the following be described in more detail with reference to the accompanying exemplary drawings, in which:
Fig. 1 shows a perspective view of a dental implant according to the invention, with a thread making means.
Figs. 2a-c depicts a cross-section of different ingrowth stages of bone tissue into an external fixture thread.
Fig. 3a shows a cross-section according to A-A or A'-A' in Fig. 3b-c.

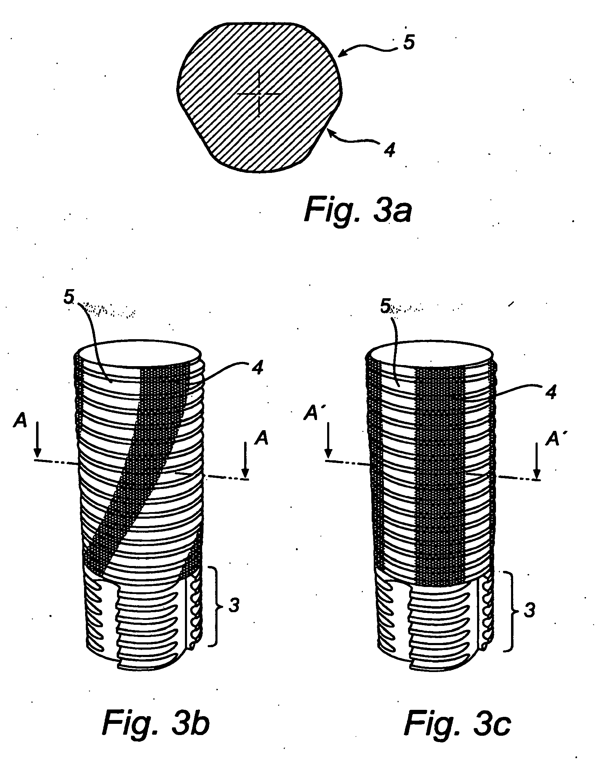

Figs. 3a-b shows in perspective view a portion of a dental implant with reduced portions.

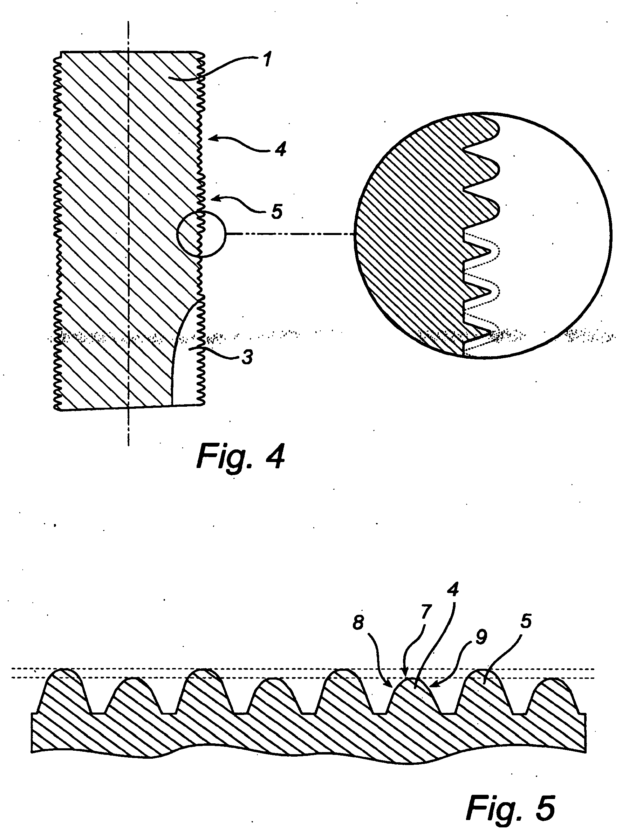

Fig. 4 shows a lengthwise cross-section view of a dental implant.
Figs. 5-7 shows various implants with differently reduced thread profiles.

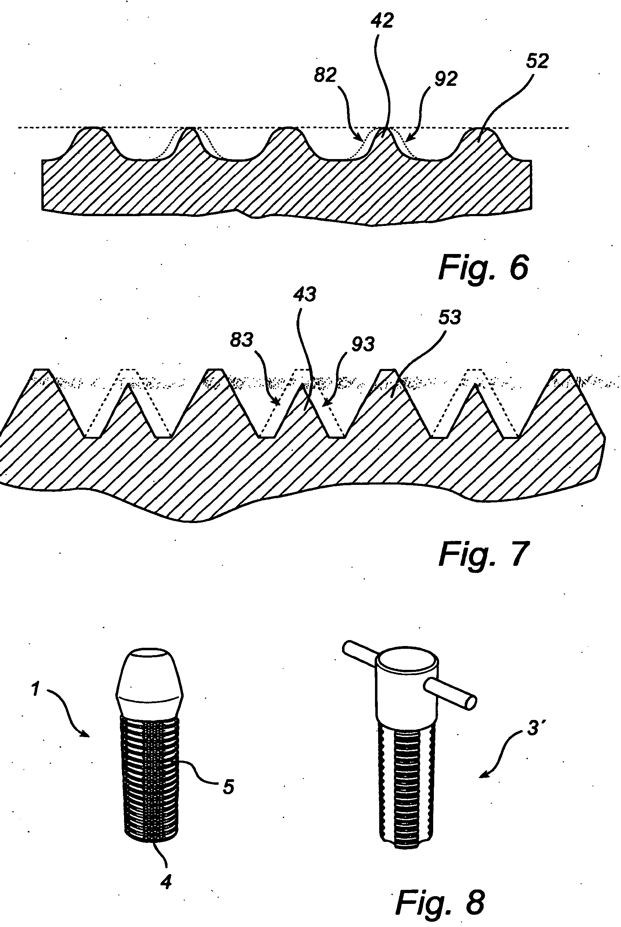

Fig. 8 shows in a perspective view an implant set according to the present invention.

### Detailed description of preferred embodiments

In the following description, reference is made to the figures which show dental implants.

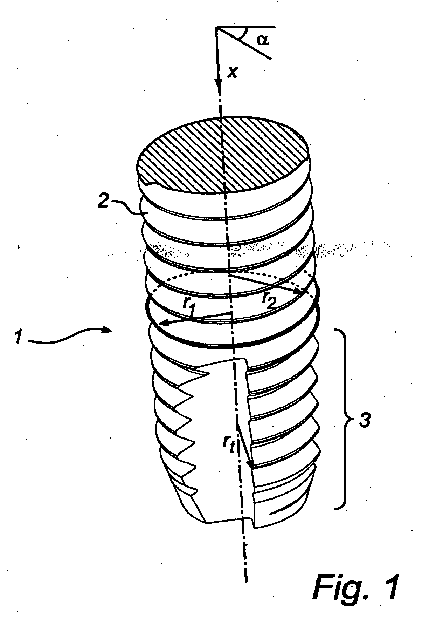

With reference to Fig. 1, there is shown a dental implant 1 provided with an external fixture thread 2 with an apical thread making means 3 having a radius rₜ, further the implant is provided with a portion of said thread having a reduced radius r2 and a portion of the thread having a radius r1 being generally equal to the radius rₜ.

The expression "radius" is here used for describing the shape of the exterior geometry of the implant 1 (or thread making means 3,3'), i.e. the radius is the distance from the centre axis of the implant 1 to the exterior surface of the thread profile, which distance is variable with the position (x) along the centre axis and the angle position (α) around the implant 1. The radius of the external fixture thread 2 is hence a variable function (f(x,α)) of x and α.

The comparison, of the radius of the first (r₁) and second (r₂) portions of the external fixture thread 2 with the radius (rₜ) of the thread making means, should be performed along a thread pitch curve (i.e. a curve along the implant 1 with constant thread pitch), since this thread pitch curve of the implant will be in contact or pass by the same point in the bone tissue 6 during screwing of the implant into the bone. This applies particularly for threads with more than one thread.

The initial part of such a thread pitch curve, i.e. the portion of the thread pitch curve associated with the thread making means 3, will form a bone thread by making a thread geometry in the bone 6 corresponding to the geometry of the radius rₜ of the thread making means 3. Thereafter, due to further screwing of the dental implant 1 into the hole in the bone, the following part of this thread pitch curve will pass this bone thread geometry formed by the corresponding portion of the thread pitch curve of the thread making means 3.

Since one thread pitch curve of the external fixture thread may pass through both first 5 and second 4 portions/areas, where the outer radius of the second portion is reduced. Alternatively there may be two (or more) external fixture threads of which one may constitute a thread pitch curve, and hence the (second) reduced portion of the external fixture thread may follow and constitute another thread pitch curve.

With reference to the inventive concept of the present invention both these given alternatives provides an initially made thread and an implant 1 with an external fixture thread 2 having a second portion/region 4 which is reduced and hence locally provides a distance between the implant and the bone thread. This distance is advantageous for growth of new bone since it is unloaded and make it possible for blood, growth hormone etc. to faster create new bone and avoid resorption.

Preferably the reduced second portion 4 is not only a small local reduction, but a reduction providing an area at the implant which is loading the bone with low or zero stress and further providing access for blood, and other bone growth stimulating substances to the current area, which accelerates the bone growth and will prevent resorption in the area.

Further, when the implant 1 is out of contact with the bone 6, at the reduced portion, the stress on the bone 6 in this area is of course substantially reduced.

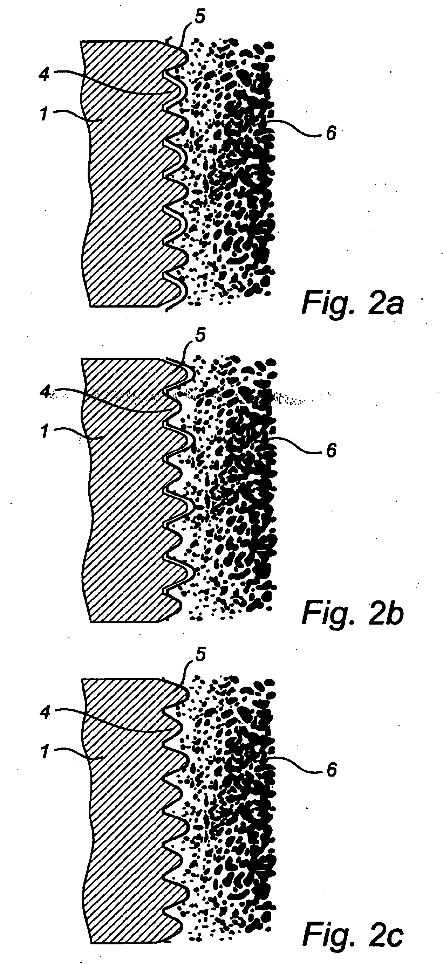

The reduced 4 and non-reduced 5 portion/s of the external fixture thread is/are positioned such that it provides a stable contact between the thread of the implant 1 and the bone thread over time. As shown in Fig. 2a, initially the non-reduced thread 5, having generally the same radius as the thread making means, will be in stable contact with the bone tissue 6 during a first period of time. After 1-4 weeks there may occur resorption in the bone area adjacent the non-reduced thread 5, as shown in Fig. 2b, on the other hand at the same time the bone tissue 6 in the area of the reduced (second) portion 4 has now grown into contact with the implant 1 and holds the implant in stable engagement. In a third stage, after about 4-8 weeks, the bone tissue 6 has grown into contact with both the reduced 4 and non-reduced 5 portions of the implant, as shown in Fig. 2c.

This alternating engagement between the reduced 4 and non-reduced 5 portions of the implant 1 and the bone 6 may be enhanced by alternating positioning of reduced 4 and non-reduced 5 portions/areas at the implant. As shown in Figs. 3b-3c (which are not part of the present invention) the reduced portions 4 may be provided as sectors around the dental implant 1.

Fig. 3a could represent both the cross-section A-A and A'-A', which shows three uniformly, distributed portions with reduced radius in sectors around the implant periphery. The change from reduced to non-reduced radius may be performed with curved shape or as a sharp change.

When using an implant 1 according to the present invention it can be installed in a cylindrical bone hole in a conventional way. Usually the hole is pre-drilled in the bone where after the bone thread is formed either with a separate thread making means 3' or with an integrated thread making means 3 with the implant.

However, the apical end of the implant may or may not be provided with reduced threads 4. If a separate thread making means 3' is used, the entire length of the implant can be used for threads with reduced radius.

When using a dental implant 1 having integrated thread making means 3 it is also possible to provide portion/s with reduced radius at the thread making apical end, which will result in the thread being formed initially by the thread making means and thereafter a sometimes undesirable pressing wedge-effect will act on the bone tissue 6 from the increasing thread radius.

If the thread making apical end 3 is provided with portion/s with reduced radius, it is desirable to position these portions coronally of the thread making means with non-reduced radius in order to minimize the risk of destructive pressure on the bone tissue 6. It is of course also possible to provide a reduced radius at a portion of the thread making means, which reduced radius will continue with the thread in the direction α along the periphery, and hence there will be no risk of overloading the bone.

For the embodiments shown in the accompanying drawings it is advantageous if about 1-50% of the area of the external fixture thread 2, intended for holding/engaging the bone 6, is reduced and the resulting portion is non-reduced. More preferably the reduced area of the external fixture thread constitutes 10-20%. According to an example which is not part of the present invention, the reduced portion 4 may be provided as two 90° sectors, alternatively totally three sectors of each 60° or even more sectors. Further, with specific alternative embodiments more than 50% might be reduced.

The desired effect will also be achieved by e.g. a double thread, of which one of the threads is reduced. This is due to that the double thread will provide alternating reduced and non-reduced areas along the implant, as seen in Figs. 2a-c.

The inventive concept according to the present invention can be applied to a number of different thread profiles. Embodiments of different thread profiles are shown in Figs. 5-7. Fig 5 shows non-reduced threads 5 and reduced threads 4, having crest 7, apical 8 and coronal 9 flanks reduced. Further, Fig. 7 shows a more sharp thread profile with reduced flanks 83,93 and reduced crest. Fig. 6 shows the principle for an implant thread having non-reduced thread profiles 52 and reduced thread profiles 42, where the apical and coronal flanks are reduced. It is also possible to provide a number of subsequently reduced threads, as shown in Fig. 4.

According to one aspect of the present invention, the thread making means is not necessarily positioned at the apical end of the implant, but at the apical end of an external fixture thread. An implant might for instance have a first portion with a maximum radius less than the radius of a thread making means coronally of said first portion, the thread making means making threads for a second coronal portion according to the present invention. Further, said first portion might have threads and its own thread making means, integral or not with the implant, also possibly according to the present invention such that threads on the first portion have reduced radius compared to the corresponding radius of the thread making means for the first portion.

## Claims

1. An implant (1), comprising:
- an external fixture thread (2) having a radius,
- thread making means (3) at an apical end of the external thread (2) for making a bone thread in bone tissue (6) during insertion of the implant,
said thread making means (3) having a radius (rₜ), which radius (rₜ) is generally equal to the radius (r₁) of first portions (5) of the external fixture thread (2), wherein the radius (r₂) of second portions (4) of said external fixture thread is reduced in relation to said radius (rₜ) of the thread making means (3) making the bone thread geometry in which said first and second portions of the external thread will pass such that initially after insertion of the implant the implant is out of contact with the bone at the reduced portions (4), wherein said portions with reduced radius (4) and said portions with non-reduced radius (5) are provided alternatingly along the length of the implant.

2. An implant according to claim 1, wherein the reduced portions (4) are provided cyclically/periodically along the length of the implant.

3. An implant (1) according to claim 1 or claim 2, wherein portions with reduced radius (4) are provided repeatedly at said implant for providing both initial stability and future stability between the implant and the bone due to the ingrowth process of the bone tissue with said implant.

4. An implant (1) according to any of the preceding claims, wherein the reduced radius in relation to said radius of the thread making means is preferably 5% or less, more preferably 2,4% or less, and most preferably 0,06-0,16% of the radius of the thread making means.

5. An implant (1) according to any of the claims 1-3, wherein the distance between a specific position on a reduced thread and the closest corresponding position on the thread making means is preferably 100µm or less, more preferably 50µm or less, and most preferably 10-30µm.

6. An implant (1) according to any of the preceding claims, wherein a crest (7) of said second portions of said external fixture thread of said implant is reduced.

7. An implant (1) according to any of the preceding claims, wherein an apical flank (8) of said second portions of said external fixture thread (2) of said implant is reduced.

8. An implant (1) according to any of the preceding claims, wherein a coronal flank (9) of said second portions of said external fixture thread (2) of said implant is reduced.

9. An implant (1) according to any of the preceding claims, wherein a bottom of said second portions of said external fixture thread (2) of said implant is reduced.

10. An implant (1) according to any of the preceding claims, wherein the reduction of said second portions of the fixture thread (2) is uniform along the axial extension of the implant (1).

11. An implant (1) according to any of the preceding claims, wherein said core of said second portion of the external fixture thread is provided with a reduced radius.

12. An implant (1) according to any one of the preceding claims, wherein the implant (1) is a dental implant.

13. An implant set, comprising:
- an implant (1),
- a separate thread making means (3') for making a bone thread in bone tissue (6) prior to insertion of said implant (1),
said implant (1) comprising an external fixture thread (2) having a radius, said thread making means having a radius (rₜ) being generally equal to the radius (r₁) of first portions (5) of said external fixture thread, wherein the radius (r₂) of second portions (4) of said external fixture thread is reduced in relation to the radius (rₜ) of said thread making means (3') making the bone thread geometry in which said first and second portions of the external thread will pass such that initially after insertion of the implant the implant is out of contact with the bone at the reduced portions (4), wherein said portions with reduced radius (4) and said portions with non-reduced radius (5) are provided alternatingly along the length of the implant.

14. An implant (1) set according to claim 13, wherein the reduced portions are provided cyclically/periodically along the length of the implant.

15. An implant (1) set according to claim 13 or claim 14, wherein the reduced radius in relation to said radius of said thread making means is preferably 5% or less, more preferably 2,4% or less, and most preferably 0,06-0,16% of the radius of the thread making means.

16. An implant set according to claim 13 or claim 14, wherein the distance between a specific position on a reduced thread and the closest corresponding position on the thread making means is preferably 100µm or less, more preferably 50µm or less, and most preferably 10-30pm.

17. An implant set according to any of the claims 13 to 16, wherein a crest (7) of said second portions of said external fixture thread of said implant (1) is reduced.

18. An implant set according to any of the claims 13 to 17, wherein an apical flank (8) of said second portions of said external fixture thread of said implant (1) is reduced.

19. An implant set according to any of the claims 13 to 18, wherein a coronal flank (9) of said second portions of said external fixture thread of said implant (1) is reduced.

20. An implant set according to any of the claims 13 to 19, wherein a bottom of said second portions of said external fixture thread of said implant (1) is reduced.

21. An implant set according to any of the claims 13 to 20, wherein the reduction of said second portions of the fixture thread (2) is uniform as seen along the axial extension of the implant (1).

22. An implant set according to any one of the claims 13 to 21, wherein the implant (1) is a dental implant.

## Patentansprüche

1. Implantat (1), umfassend:
- ein äußeres Befestigungsgewinde (2) mit einem Radius,
- Mittel zur Gewindeherstellung (3) an einem apikalen Ende des äußeren Gewindes (2) zur Herstellung eines Knochengewindes in Knochengewebe (6) während des Einsetzens des Implantats,
wobei die Mittel zur Gewindeherstellung (3) einen Radius (rₜ) aufweisen, wobei dieser Radius (rₜ) im Allgemeinen gleich dem Radius (r₁) erster Abschnitte (5) des äußeren Befestigungsgewindes (2) ist, wobei der Radius (r₂) zweiter Abschnitte (4) des äußeren Befestigungsgewindes im Verhältnis zu dem Radius (rₜ) der Mittel zur Gewindeherstellung (3) reduziert ist, wodurch bewirkt wird, dass die Geometrie des Knochengewindes, in welche die ersten und zweiten Abschnitte des äußeren Gewindes dann gelangen, so beschaffen ist, dass nach dem Einsetzen des Implantats das Implantat sich zunächst an den reduzierten Abschnitten (4) nicht in Kontakt mit dem Knochen befindet, wobei die Abschnitte mit reduziertem Radius (4) und die Abschnitte mit nicht reduziertem Radius (5) abwechselnd entlang der Länge des Implantats vorgesehen sind.

2. Implantat nach Anspruch 1, wobei die reduzierten Abschnitte (4) zyklisch/periodisch entlang der Länge des Implantats vorgesehen sind.

3. Implantat (1) nach Anspruch 1 oder Anspruch 2, wobei Abschnitte mit reduziertem Radius (4) wiederholt an dem Implantat vorgesehen sind, um sowohl anfängliche Stabilität als auch zukünftige Stabilität zwischen dem Implantat und dem Knochen infolge des Prozesses des Einwachsens des Knochengewebes in das Implantat zu gewährleisten.

4. Implantat (1) nach einem der vorhergehenden Ansprüche, wobei der reduzierte Radius im Verhältnis zu dem Radius der Mittel zur Gewindeherstellung vorzugsweise 5 % oder weniger, stärker bevorzugt 2,4 % oder weniger und am stärksten bevorzugt 0,06-0,16 % des Radius der Mittel zur Gewindeherstellung beträgt.

5. Implantat (1) nach einem der Ansprüche 1-3, wobei der Abstand zwischen einer spezifischen Position auf einem reduzierten Gewinde und der am nächsten befindlichen entsprechenden Position auf den Mitteln zur Gewindeherstellung vorzugsweise 100 µm oder weniger, stärker bevorzugt 50 µm oder weniger und am stärksten bevorzugt 10-30 µm beträgt.

6. Implantat (1) nach einem der vorhergehenden Ansprüche, wobei eine Spitze (7) der zweiten Abschnitte des äußeren Befestigungsgewindes des Implantats reduziert ist.

7. Implantat (1) nach einem der vorhergehenden Ansprüche, wobei eine apikale Flanke (8) der zweiten Abschnitte des äußeren Befestigungsgewindes (2) des Implantats reduziert ist.

8. Implantat (1) nach einem der vorhergehenden Ansprüche, wobei eine koronale Flanke (9) der zweiten Abschnitte des äußeren Befestigungsgewindes (2) des Implantats reduziert ist.

9. Implantat (1) nach einem der vorhergehenden Ansprüche, wobei ein Boden der zweiten Abschnitte des äußeren Befestigungsgewindes (2) des Implantats reduziert ist.

10. Implantat (1) nach einem der vorhergehenden Ansprüche, wobei die Reduktion der zweiten Abschnitte des Befestigungsgewindes (2) gleichmäßig entlang der axialen Erstreckung des Implantats (1) ist.

11. Implantat (1) nach einem der vorhergehenden Ansprüche, wobei der Kern der zweiten Abschnitte des äußeren Befestigungsgewindes mit einem reduzierten Radius versehen ist.

12. Implantat (1) nach einem der vorhergehenden Ansprüche, wobei das Implantat (1) ein Dentalimplantat ist.

13. Implantatset, umfassend:
- ein Implantat (1),
- ein separates Mittel zur Gewindeherstellung (3') zur Herstellung eines Knochengewindes in Knochengewebe (6) vor dem Einsetzen des Implantats (1),
wobei das Implantat (1) ein äußeres Befestigungsgewinde (2) mit einem Radius umfasst, wobei das Mittel zur Gewindeherstellung einen Radius (rₜ) aufweist, der im Allgemeinen gleich dem Radius (r₁) erster Abschnitte (5) des äußeren Befestigungsgewindes ist, wobei der Radius (r₂) zweiter Abschnitte (4) des äußeren Befestigungsgewindes im Verhältnis zu dem Radius (rₜ) des Mittels zur Gewindeherstellung (3') reduziert ist, wodurch bewirkt wird, dass die Geometrie des Knochengewindes, in welche die ersten und zweiten Abschnitte des äußeren Gewindes dann gelangen, so beschaffen ist, dass nach dem Einsetzen des Implantats das Implantat sich zunächst an den reduzierten Abschnitten (4) nicht in Kontakt mit dem Knochen befindet, wobei die Abschnitte mit reduziertem Radius (4) und die Abschnitte mit nicht reduziertem Radius (5) abwechselnd entlang der Länge des Implantats vorgesehen sind.

14. Implantatset (1) nach Anspruch 13, wobei die reduzierten Abschnitte zyklisch/periodisch entlang der Länge des Implantats vorgesehen sind.

15. Implantatset (1) nach Anspruch 13 oder Anspruch 14, wobei der reduzierte Radius im Verhältnis zu dem Radius des Mittels zur Gewindeherstellung vorzugsweise 5 % oder weniger, stärker bevorzugt 2,4 % oder weniger und am stärksten bevorzugt 0,06-0,16 % des Radius der Mittel zur Gewindeherstellung beträgt.

16. Implantatset nach Anspruch 13 oder Anspruch 14, wobei der Abstand zwischen einer spezifischen Position auf einem reduzierten Gewinde und der am nächsten befindlichen entsprechenden Position auf dem Mittel zur Gewindeherstellung vorzugsweise 100 µm oder weniger, stärker bevorzugt 50 µm oder weniger und am stärksten bevorzugt 10-30 µm beträgt.

17. Implantatset nach einem der Ansprüche 13 bis 16, wobei eine Spitze (7) der zweiten Abschnitte des äußeren Befestigungsgewindes des Implantats (1) reduziert ist.

18. Implantatset nach einem der Ansprüche 13 bis 17, wobei eine apikale Flanke (8) der zweiten Abschnitte des äußeren Befestigungsgewindes des Implantats (1) reduziert ist.

19. Implantatset nach einem der Ansprüche 13 bis 18, wobei eine koronale Flanke (9) der zweiten Abschnitte des äußeren Befestigungsgewindes des Implantats (1) reduziert ist.

20. Implantatset nach einem der Ansprüche 13 bis 19, wobei ein Boden der zweiten Abschnitte des äußeren Befestigungsgewindes des Implantats (1) reduziert ist.

21. Implantatset nach einem der Ansprüche 13 bis 20, wobei die Reduktion der zweiten Abschnitte des Befestigungsgewindes (2) gleichmäßig entlang der axialen Erstreckung des Implantats (1) ist.

22. Implantatset nach einem der Ansprüche 13 bis 21, wobei das Implantat (1) ein Dentalimplantat ist.

## Revendications

1. Implant (1), selon :
- un filetage de fixation externe (2) ayant un rayon,
- un moyen réalisant un filetage (3) à une extrémité apicale du filetage externe (2) pour effectuer un filetage osseux dans le tissu osseux (6) pendant l'insertion de l'implant,
ledit moyen réalisant un filetage (3) ayant un rayon (rₜ), lequel rayon (rₜ) est généralement égal au rayon (r₁) de premières parties (5) du filetage externe (2), dans lequel le rayon (r₂) de deuxièmes parties (4) dudit filetage de fixation externe est réduit par rapport audit rayon (rₜ) du moyen réalisant un filetage (3), rendant telle la géométrie du filetage osseux dans laquelle passeront lesdites première et deuxième parties du filetage externe, qu'initialement après l'insertion de l'implant, l'implant ne soit pas en contact avec l'os sur les parties réduites (4), dans lequel lesdites parties au rayon réduit (4) et lesdites parties au rayon non réduit (5) sont prévues alternativement sur la longueur de l'implant.

2. Implant (1) selon la revendication 1, dans lequel les parties réduites (4) sont prévues cycliquement/périodiquement sur la longueur de l'implant.

3. Implant (1) selon la revendication 1 ou 2, dans lequel les parties au rayon réduit (4) sont prévues de manière répétée sur ledit implant pour fournir à la fois une stabilité initiale et une stabilité future entre l'implant et l'os en raison du processus de croissance du tissu osseux avec ledit implant.

4. Implant (1) selon l'une quelconque des revendications précédentes, dans lequel le rayon réduit par rapport audit rayon du moyen réalisant un filetage est de manière préférée de 5 % ou moins, de manière plus préférée de 2,4 % ou moins et de manière la plus préférée de 0,06-0,16% du rayon du moyen de réalisation d'un filetage.

5. Implant (1) selon l'une quelconque des revendications 1 à 3, dans lequel la distance entre une position spécifique sur un filetage réduit et la position correspondante la plus proche sur le moyen réalisant un filetage est de manière préférée de 100µm ou moins, de manière plus préférée de 50µm ou moins et de manière la plus préférée de 10-30µm.

6. Implant (1) selon l'une quelconque des revendications précédentes, dans lequel une crête (7) desdites deuxièmes parties dudit filetage de fixation externe dudit implant (1) est réduite.

7. Implant (1) selon l'une quelconque des revendications précédentes, dans lequel un flanc apical (8) desdites deuxièmes parties dudit filetage de fixation externe (2) dudit implant est réduit.

8. Implant (1) selon l'une quelconque des revendications précédentes, dans lequel un flanc coronaire (9) desdites deuxièmes parties dudit filetage de fixation externe (2) dudit implant est réduit.

9. Implant (1) selon l'une quelconque des revendications précédentes, dans lequel un fond desdites deuxièmes parties dudit filetage de fixation externe (2) dudit implant est réduit.

10. Implant (1) selon l'une quelconque des revendications précédentes, dans lequel la réduction desdites deuxièmes parties dudit filetage de fixation externe (2) est uniforme le long de l'extension axiale de l'implant (1).

11. Implant (1) selon l'une quelconque des revendications précédentes, dans lequel ledit noyau de ladite deuxième partie du filetage de fixation externe est munie d'un rayon réduit.

12. Implant (1) selon l'une quelconque des revendications précédentes, dans lequel l'implant (1) est un implant dentaire.

13. Kit d'implant, comprenant :
- un implant (1),
- un moyen réalisant un filetage séparé (3') pour effectuer un filetage osseux dans un tissu osseux (6) avant l'insertion dudit implant (1),
ledit implant (1) comprenant un filetage de fixation externe (2) ayant un rayon, ledit moyen réalisant un filetage ayant un rayon (rₜ) généralement égal au rayon (r₁) de premières parties (5) dudit filetage de fixation externe, dans lequel le rayon (r₂) de deuxièmes parties (4) dudit filetage de fixation externe est réduit par rapport audit rayon (rₜ) dudit moyen réalisant un filetage (3'), rendant telle la géométrie du filetage osseux dans laquelle passeront lesdites première et deuxième parties du filetage externe, qu'initialement après l'insertion de l'implant, l'implant ne soit pas en contact avec l'os sur les parties réduites (4), dans lequel lesdites parties au rayon réduit (4) et lesdites parties au rayon non réduit (5) sont prévues alternativement sur la longueur de l'implant.

14. Kit d'implant (1) selon la revendication 13, dans lequel les parties réduites sont prévues cycliquement/périodiquement sur la longueur de l'implant.

15. Kit d'implant (1) selon la revendication 13 ou 14, dans lequel le rayon réduit par rapport audit rayon du moyen réalisant un filetage est de manière préférée de 5 % ou moins, de manière plus préférée de 2,4 % ou moins et de manière la plus préférée de 0,06-0,16% du rayon du moyen de réalisation d'un filetage.

16. Kit d'implant selon la revendication 13 ou 14, dans lequel la distance entre une position spécifique sur un filetage réduit et la position correspondante la plus proche sur le moyen réalisant un filetage est de manière préférée de 100µm ou moins, de manière plus préférée de 50µm ou moins et de manière la plus préférée de 10-30µm.

17. Kit d'implant selon l'une quelconque des revendications 13 à 16, dans lequel une crête (7) desdites deuxièmes parties dudit filetage de fixation externe dudit implant (1) est réduite.

18. Kit d'implant selon l'une quelconque des revendications 13 à 17, dans lequel un flanc apical (8) desdites deuxièmes parties dudit filetage de fixation externe dudit implant (1) est réduit.

19. Kit d'implant selon l'une quelconque des revendications 13 à 18, dans lequel un flanc coronaire (9) desdites deuxièmes parties dudit filetage de fixation externe dudit implant (1) est réduit.

20. Kit d'implant selon l'une quelconque des revendications 13 à 19, dans lequel un fond desdites deuxièmes parties dudit filetage de fixation externe dudit implant (1) est réduit.

21. Kit d'implant selon l'une quelconque des revendications 13 à 20, dans lequel la réduction desdites deuxièmes parties dudit filetage de fixation externe (2) est uniforme le long de l'extension axiale de l'implant (1).

22. Kit d'implant selon l'une quelconque des revendications 13 à 21, dans lequel l'implant (1) est un implant dentaire.
